(19)

**Europäisches Patentamt**
**European Patent Office**
**Office européen des brevets**

(11) **EP 4 652 987 A1**

(12) **EUROPEAN PATENT APPLICATION**
published in accordance with Art. 153(4) EPC

(43) Date of publication:
26.11.2025 Bulletin 2025/48

(21) Application number: 24829699.8

(22) Date of filing: 03.01.2024

(51) International Patent Classification (IPC):
A61K 9/06 (2006.01)    A61K 38/18 (2006.01)
A61K 47/18 (2017.01)    A61P 17/02 (2006.01)

(86) International application number:
PCT/CN2024/070258

(87) International publication number:
WO 2025/001036 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.06.2023 CN 202310760678

(71) Applicant: Shanghai Tenry Pharmaceutical Co.,
Ltd.
Fengxian District, Shanghai 201400 (CN)

(72) Inventors:
• ZHU, Guoxing
Shanghai 201400 (CN)

• FU, Dongjun
Shanghai 201400 (CN)
• TANG, Fang
Shanghai 201400 (CN)
• GUAN, Lufan
Shanghai 201400 (CN)
• LI, Zhengwu
Shanghai 201400 (CN)
• HE, Jiaxin
Shanghai 201400 (CN)

(74) Representative: ABG Intellectual Property Law,
S.L.
Avenida de Burgos, 16D
Edificio Euromor
28036 Madrid (ES)

(54) **RECOMBINANT HUMAN ACIDIC FIBROBLAST GROWTH FACTOR GEL AND PREPARATION PROCESS THEREFOR**

(57)    A recombinant human acidic fibroblast growth factor (aFGF) gel. The gel comprises a gel matrix, a thickener, and a protectant. The gel matrix is selected from one or more of sodium carboxymethyl cellulose, hypromellose, sodium alginate, sodium polyacrylate, sodium hyaluronate, polyethylene glycol, and gelatin. The gel thickener is selected from polyvinyl alcohol. The protectant is selected from a combination of histidine and low-molecular-weight heparin sodium. A combination of the gel matrix and the thickener (polyvinyl alcohol) is used, and on this basis, histidine and low-molecular-weight heparin sodium are used as a protein protectant, so that these auxiliary materials synergistically improve the stability of the recombinant human acidic fibroblast growth factor (aFGF) gel. It is found through studies that the combination of histidine and heparin sodium can serve as an alternative protein protectant to human serum albumin and can still achieve a good protection effect at a low concentration, thereby effectively reducing the production costs.

EP 4 652 987 A1

**Description**

**Technical Field**

The present invention belongs to the field of pharmaceutical preparations, and particularly relates to a recombinant human acidic fibroblast growth factor gel and a preparation process therefor.

**Background Art**

An acidic fibroblast growth factor (aFGF) is a member of the fibroblast growth factor (FGF) family that is derived from mesoderm and neuroectoderm. It is a multifunctional cell growth factor and a trace active substance with a wide range of mitogenic effects. It is mainly distributed in organs or tissues such as the brain, pituitary gland, nervous tissue, retina, adrenal gland, heart, and bone, with very low concentrations in other tissues and extremely low concentrations in serum and body fluids. It is an endogenous active factor that is closely related to the healing process of tissue trauma, and has a significant promoting effect on tissue trauma healing, nerve damage repair and vascular regeneration. Such factor is used for the following wounds: deep II degree burn wounds, chronic ulcer wounds (including residual wounds after trauma, diabetic ulcers, vascular ulcers and bedsores), to promote wound healing.

Common dosage forms of acidic fibroblast growth factor (aFGF) include lyophilized powder, eye drops and gels. For lyophilized powder, the solvent provided in the package needs to be poured into the bottle containing rhaFGF lyophilized powder, and after the sprayer head provided in the package is capped (caught), it can be used. When used, the liquid is directly sprayed on the wound after debridement. Eye drops and gels can be used directly. Gels incorporate the characteristics of polymer materials, and can effectively prolong the retention time of added active factors in specific sites, improve drug absorption and bioavailability, have targeted functions, and can slowly release drugs, which can reduce the number of clinical drug administrations and is convenient to use, offering broad market competitiveness.

Gels have the advantages of being easy to spread, comfortable, non-greasy, easy to wash off, able to absorb tissue exudate, not interfering with the normal physiological function of the skin, having a certain water-retaining effect, and promoting the transdermal absorption of drugs. However, acidic fibroblast growth factor (aFGF) aqueous gels are sensitive to external conditions such as temperature, and are prone to denaturation and inactivation when left at room temperature for a long time. In order to solve the above-mentioned technical problems, Chinese invention patent application 202111189805.1 uses Carbomer 940 as a gel matrix, and human serum albumin, sodium heparin, etc. as protective agents to obtain a recombinant human acidic fibroblast growth factor (aFGF) gel that is insensitive to temperature conditions. Although this patent application solves the problem of poor stability of acidic fibroblast growth factor (aFGF) aqueous gels, the use of a large amount of protective agents will undoubtedly increase the cost of the product.

**Summary of the Invention**

In view of the deficiencies of the prior art, the present invention provides a new recombinant human acidic fibroblast growth factor (aFGF) gel to solve the problems of poor product stability and high preparation cost in the prior art.

Specifically, the present invention provides a recombinant human acidic fibroblast growth factor (aFGF) gel, which comprises a gel matrix, a thickener and a protectant, and the gel matrix is selected from one or more of sodium carboxymethyl cellulose, hypromellose, sodium alginate, sodium polyacrylate, sodium hyaluronate, polyethylene glycol, and gelatin, the gel thickener is selected from polyvinyl alcohol, and the protectant is selected from a combination of histidine and heparin sodium.

Further, the present invention provides a recombinant human acidic fibroblast growth factor (aFGF) gel, wherein the protectant is selected from a combination of histidine and heparin sodium, and the heparin sodium is selected from low-molecular-weight heparin sodium, and preferably, the average molecular weight of the low-molecular-weight heparin sodium is 3000 to 8000.

Further, the present invention provides a recombinant human acidic fibroblast growth factor (aFGF) gel, wherein the mass of the histidine is 0.8 to 2‰ of the mass of the gel, preferably 1 to 1.6‰; the mass of the heparin sodium is 0.05 to 1‰ of the mass of the gel, preferably 0.08 to 0.2‰.

Further, the present invention provides a recombinant human acidic fibroblast growth factor (aFGF) gel, wherein the mass of the gel matrix is 0.5 to 5% of the mass of the gel, preferably 1 to 3%.

Further, the present invention provides a recombinant human acidic fibroblast growth factor (aFGF) gel, wherein the mass of the thickener is 0.5 to 3% of the mass of the gel, preferably 1 to 2%.

Further, the present invention provides a recombinant human acidic fibroblast growth factor (aFGF) gel, which also contains a buffer, and the buffer salt is selected from the group consisting of phosphate buffer system, citrate buffer system, and Tris buffer system, preferably phosphate buffer system, and the preferred pH value of the buffer salt is 6.0 to 7.0, the concentration is 0.01 to 0.05 mol/L, and the preferred concentration is 0.02 mol/L.

Further, the present invention provides a recombinant human acidic fibroblast growth factor (aFGF) gel, wherein the relative amount of the recombinant human acidic fibroblast growth factor in the gel is $2\times10^3$ to $8\times10^3$ IU/g.

Further, the present invention provides a recombinant human acidic fibroblast growth factor (aFGF) gel, which also contains a humectant and a preservative, preferably the humectant is selected from glycerols; the preservative is selected from p-hydroxybenzoate preservatives, preferably the preservative is selected from one or more of methyl p-hydroxybenzoate, ethyl p-hydroxybenzoate, propyl p-hydroxybenzoate, and isopropyl p-hydroxybenzoate; the humectant also has a solubility aid effect.

Further, the present invention provides a recombinant human acidic fibroblast growth factor (aFGF) gel, wherein the mass of the preservative is 0.1 to 3‰ of the mass of the gel, preferably 0.2 to 1‰; and the mass of the humectant is 0.2 to 4% of the mass of the gel, preferably 0.5 to 2%.

The present invention has the following beneficial effects:

The present invention selects a combination of a gel matrix and a thickener (polyvinyl alcohol); on this basis, histidine and heparin sodium are selected as protein protectants. The above auxiliary materials synergistically improve the stability of the recombinant human acidic fibroblast growth factor (aFGF) gel, and the prepared gel product still maintains high stability and relative activity under high temperature conditions.

After screening, it was determined that the combination of histidine and heparin sodium can be used as an alternative protein protectant for human albumin. It can still achieve a high protection effect under lower concentration conditions, effectively reducing production costs.

## Detailed Description of Examples

The present invention is described in detail and specifically by specific examples below to provide a better understanding of the present invention, but the following examples do not limit the scope of the present invention.

The phosphate buffer described in the present invention is $Na_2HPO_4$, $NaH_2PO_4 \cdot H_2O$ buffer, with a concentration of 0.02 mol/L and a pH value of 6.0 to 7.0; the weighing of reagents and the preparation of the formulation are performed under sterile conditions; the average molecular weight of the low-molecular-weight heparin sodium in the examples of the present invention is 3000 to 8000.

**Example 1:** Taking the preparation of 100 g of recombinant human acidic fibroblast growth factor (aFGF) gel as an example, the specific prescription and preparation method are as follows:

| | |
|---|---|
| rhaFGF | $4\times10^5$ IU |
| Sodium carboxymethyl cellulose | 2 g |
| Polyvinyl alcohol | 1 g |
| Histidine | 0.1 g |
| Low-molecular-weight heparin sodium | 0.02 g |
| Ethyl p-hydroxybenzoate | 0.05 g |
| Glycerol | 2 g |
| $NaH_2PO_4 \cdot H_2O$ | 0.189 g |
| $Na_2HPO_4$ | 0.089 g |

(1) Take about 40 ml of water for injection, add the prescribed amount of preservatives and humectants, heat to dissolve and then add the prescribed amount of gel matrix. After fully swelling overnight, sterilize with high-pressure wet heat at 121°C for 30 minutes, let stand and cool to room temperature, and obtain solution A for use.

(2) Add the prescribed amount of $Na_2HPO_4$, $NaH_2PO_4 \cdot H_2O$, thickener and protectant into 50 ml of water for injection, respectively, stir to dissolve and then add the prescribed amount of recombinant human acidic fibroblast growth factor stock solution, mix thoroughly and obtain solution B for use.

(3) Filter solution B through a 0.22 $\mu$m filter membrane to sterilize it and then add it to solution A. Add the remaining amount of water for injection to make up to 100 g. Stir thoroughly and let stand to obtain rhaFGF gel.

**Example 2:** Taking the preparation of 100 g of recombinant human acidic fibroblast growth factor (aFGF) gel as an example, the specific prescription is as follows:

| | |
|---|---|
| rhaFGF | $6\times10^5$ IU |

(continued)

| | |
|---|---|
| Hypromellose | 2.5 g |
| Polyvinyl alcohol | 1 g |
| Histidine | 0.1 g |
| Low-molecular-weight heparin sodium | 0.02 g |
| Methyl p-hydroxybenzoate | 0.05 g |
| Glycerol | 2 g |
| $NaH_2PO_4 \cdot H_2O$ | 0.189 g |
| $Na_2HPO_4$ | 0.089 g |

The preparation method is as in Example 1.

**Example 3:** Taking the preparation of 100 g of recombinant human acidic fibroblast growth factor (aFGF) gel as an example, the specific prescription is as follows:

| | |
|---|---|
| rhaFGF | $4 \times 10^5$ IU |
| Sodium alginate | 4 g |
| Polyvinyl alcohol | 1.5 g |
| Histidine | 0.1 g |
| Low-molecular-weight heparin sodium | 0.02 g |
| Propyl p-hydroxybenzoate | 0.05 g |
| Glycerol | 2 g |
| $NaH_2PO_4 \cdot H_2O$ | 0.189 g |
| $Na_2HPO_4$ | 0.089 g |

**Comparative Example 1:** Taking the preparation of 100 g of recombinant human acidic fibroblast growth factor (aFGF) gel as an example, the specific prescription is as follows:

| | |
|---|---|
| rhaFGF | $4 \times 10^5$ IU |
| Sodium carboxymethyl cellulose | 2 g |
| Histidine | 0.1 g |
| Low-molecular-weight heparin sodium | 0.02 g |
| Ethyl p-hydroxybenzoate | 0.05 g |
| Glycerol | 2 g |
| $NaH_2PO_4 \cdot H_2O$ | 0.189 g |
| $Na_2HPO_4$ | 0.089 g |

The preparation method is as in Example 1.

**Comparative Example 2:** Taking the preparation of 100 g of recombinant human acidic fibroblast growth factor (aFGF) gel as an example, the specific prescription is as follows:

| | |
|---|---|
| rhaFGF | $4 \times 10^5$ IU |
| Sodium carboxymethyl cellulose | 2 g |
| Polyvinyl alcohol | 1 g |
| Low-molecular-weight heparin sodium | 0.02 g |
| Ethyl p-hydroxybenzoate | 0.05 g |
| Glycerol | 2 g |
| $NaH_2PO_4 \cdot H_2O$ | 0.189 g |
| $Na_2HPO_4$ | 0.089 g |

The preparation method is as in Example 1.

**Comparative Example 3:** Taking the preparation of 100 g of recombinant human acidic fibroblast growth factor (aFGF) gel as an example, the specific prescription is as follows:

| | |
|---|---|
| rhaFGF | $4 \times 10^5$ IU |
| Sodium carboxymethyl cellulose | 2 g |
| Polyvinyl alcohol | 1 g |
| Histidine | 0.1 g |
| Ethyl p-hydroxybenzoate | 0.02 g |
| Glycerol | 2 g |
| $NaH_2PO_4 \cdot H_2O$ | 0.189 g |
| $Na_2HPO_4$ | 0.089 g |

The prepared rhAFGF gel was subjected to the following experimental verification, and the test results were shown in Table 1.

(1) rhAFGF gel stability test

Take 5 g of the gels of Examples 1-3 and Comparative Examples 1-3, seal them in aluminum tubes, and store them at 25°C and 40°C, respectively. Check the appearance of the gel regularly, take samples to determine the activity of rhaFGF in the gel, and calculate the relative activity value.

(2) rhAFGF activity assay (using MTT method)

After re-dissolving the rhAFGF activity assay working standard, use the test culture medium to make three 16-fold dilutions and then seven 4-fold dilutions. Load the last 8 dilution gradients for detection in duplicate wells. After re-dissolving the test sample according to the indicated amount, use the test culture medium to make three 16-fold dilutions and then seven 4-fold dilutions. Load the last 8 dilution gradients for detection in duplicate wells. Complete culture medium was used for incubation and passage of 3T3 cells. 3T3 cells in the logarithmic growth phase were fully digested with trypsin into individual cells and collected, the cell density was adjusted to about $1.0 \times 10^5$ cells/ml with test culture medium to prepare a cell suspension. The cell suspension was inoculated into a 96-well plate with samples in 50 ul per well, and incubated in 37°C incubator containing 5% $CO_2$ for 66 to 72 hours. 10 ul MTT solution was added to each well, which was incubated in 37°C incubator containing 5% $CO_2$ for 4 to 6 hours. 100ul 10% SDS was added to each well, which was incubated in 37°C incubator containing 5% $CO_2$ overnight. Colorimetry was performed on an ELISA reader at a wavelength of 570nm or 570nm/630nm. The OD value was read and recorded, and the corresponding OD-rhAFGF concentration curve was made. Its activity was calculated according to the following formula:

$$\text{Sample biological activity (U/ml)} = Pr \times Ds \times Es / (Dr \times Er)$$

Pr is the biological activity of the standard, in U/ml; and Ds is the pre-dilution factor of the test sample.
Dr is the pre-dilution factor of the standard; and Es is the dilution factor of the test sample equivalent to the half-effective dose of the standard.
Er is the dilution factor of the half-effective dose of the standard.

Table 1: Relative activity results of stability study at 25°C (relative activity at day 0 is 100%)

| Example/Comparative Example | Time (day) | Appearance | pH value | relative activity |
|---|---|---|---|---|
| Example 1 | 10 | meet the | 6.5 | 104.1% |
| | | requirement | | |
| | 20 | meet the requirement | 6.6 | 99.3% |
| | 30 | meet the requirement | 6.4 | 97.1% |
| | 60 | meet the requirement | 6.5 | 99.4% |
| | 90 | meet the requirement | 6.6 | 100.9% |

(continued)

| Example/Comparative Example | Time (day) | Appearance | pH value | relative activity |
|---|---|---|---|---|
| Example 2 | 10 | meet the requirement | 6.5 | 101.1% |
| | 20 | meet the requirement | 6.6 | 103.7% |
| | 30 | meet the requirement | 6.4 | 99.3% |
| | 60 | meet the requirement | 6.5 | 98.6% |
| | 90 | meet the requirement | 6.6 | 102.1% |
| Example 3 | 10 | meet the requirement | 6.6 | 99.4% |
| | 20 | meet the requirement | 6.5 | 104.8% |
| | 30 | meet the requirement | 6.4 | 101.2% |
| | 60 | meet the requirement | 6.5 | 97.8% |
| | 90 | meet the requirement | 6.4 | 99.2% |
| Comparative Example 1 | 10 | meet the requirement | 6.5 | 97.1% |
| | 20 | meet the requirement | 6.5 | 84.9% |
| | 30 | meet the requirement | 6.6 | 76.9% |
| | 60 | meet the requirement | 6.5 | 69.4% |
| | 90 | meet the requirement | 6.4 | 57.6% |
| Comparative Example 2 | 10 | meet the requirement | 6.6 | 100.8% |
| | 20 | meet the requirement | 6.5 | 96.7% |
| | 30 | meet the requirement | 6.7 | 88.4% |
| | 60 | meet the requirement | 6.5 | 80.2% |
| | 90 | meet the requirement | 6.4 | 76.2% |
| Comparative Example 3 | 10 | meet the requirement | 6.5 | 92.1% |
| | 20 | meet the requirement | 6.6 | 81.7% |
| | 30 | meet the requirement | 6.5 | 73.2% |
| | 60 | meet the requirement | 6.4 | 57.5% |
| | 90 | meet the requirement | 6.5 | 38.6% |

Table 2: Relative activity results of stability study at 40°C (relative activity at day 0 is 100%)

| Example/Comparative Example | Time (day) | Appearance | pH value | relative activity |
|---|---|---|---|---|
| Example 1 | 10 | meet the requirement | 6.6 | 103.1% |
| | 20 | meet the requirement | 6.5 | 97.3% |
| | 30 | meet the requirement | 6.7 | 98.1% |
| | 60 | meet the requirement | 6.5 | 99.4% |
| | 90 | meet the requirement | 6.4 | 97.9% |
| Example 2 | 10 | meet the requirement | 6.5 | 99.4% |
| | 20 | meet the requirement | 6.5 | 103.2% |
| | 30 | meet the requirement | 6.4 | 100.8% |
| | 60 | meet the requirement | 6.5 | 96.8% |
| | 90 | meet the requirement | 6.6 | 99.2% |

(continued)

| Example/Comparative Example | Time (day) | Appearance | pH value | relative activity |
|---|---|---|---|---|
| Example 3 | 10 | meet the requirement | 6.5 | 97.4% |
| | 20 | meet the requirement | 6.5 | 99.8% |
| | 30 | meet the requirement | 6.4 | 100.2% |
| | 60 | meet the requirement | 6.5 | 96.9% |
| | 90 | meet the requirement | 6.6 | 98.4% |
| Comparative Example 1 | 10 | meet the requirement | 6.6 | 104.1% |
| | 20 | meet the requirement | 6.5 | 88.1% |
| | 30 | meet the requirement | 6.4 | 77.7% |
| | 60 | meet the requirement | 6.5 | 70.1% |
| | 90 | meet the requirement | 6.4 | 58.4% |
| Comparative Example 2 | 10 | meet the requirement | 6.5 | 96.6% |
| | 20 | meet the requirement | 6.4 | 82.4% |
| | 30 | meet the requirement | 6.5 | 72.1% |
| | 60 | meet the requirement | 6.6 | 58.8% |
| | 90 | meet the requirement | 6.5 | 49.6% |
| Comparative Example 3 | 10 | meet the requirement | 6.7 | 82.3% |
| | 20 | meet the requirement | 6.6 | 67.3% |
| | 30 | meet the requirement | 6.5 | 43.5% |
| | 60 | meet the requirement | 6.5 | 26.2% |
| | 90 | meet the requirement | 6.5 | 14.6% |

The above contents cannot be used to determine that the specific implementation of the present invention is limited to these descriptions. Those skilled in the art to which the present invention belongs can make several simple deductions or substitutions without departing from the concept of the present invention, which should be regarded as belonging to the protection scope of the patent of the present invention determined by the submitted claims.

**Claims**

1. A recombinant human acidic fibroblast growth factor (aFGF) gel, **characterized in that** the gel comprises a gel matrix, a thickener, and a protectant, and the gel matrix is selected from one or more of sodium carboxymethyl cellulose, hypromellose, sodium alginate, sodium polyacrylate, sodium hyaluronate, polyethylene glycol, and gelatin, the gel thickener is selected from polyvinyl alcohol, and the protectant is selected from a combination of histidine and low-molecular-weight heparin sodium; preferably, the average molecular weight of the low-molecular-weight heparin sodium is 3000 to 8000.

2. The recombinant human acidic fibroblast growth factor (aFGF) gel according to claim 1, **characterized in that** the mass of the gel matrix is 0.5 to 5.0% of the mass of the gel, preferably 1.0 to 3.0%.

3. The recombinant human acidic fibroblast growth factor (aFGF) gel according to claim 1, **characterized in that** the mass of the thickener is 0.5 to 3.0% of the mass of the gel, preferably 1 to 2%.

4. The recombinant human acidic fibroblast growth factor (aFGF) gel according to claim 1, **characterized in that** the mass of the histidine is 0.8 to 2‰ of the mass of the gel, preferably 1 to 1.6‰; and the mass of the low-molecular-weight heparin sodium is 0.05 to 1‰ of the mass of the gel, preferably 0.08 to 0.2‰.

5. The recombinant human acidic fibroblast growth factor (aFGF) gel according to claim 1, **characterized in that** the gel further comprises a buffer, the buffer salt is selected from the group consisting of phosphate buffer system, citrate buffer system, and Tris buffer system, preferably phosphate buffer system, and a preferred pH value of the buffer salt is 6.0 to 7.0, a concentration is 0.01 to 0.05 mol/L, and a preferred concentration is 0.02 mol/L.

6. The recombinant human acidic fibroblast growth factor (aFGF) gel according to claim 1, **characterized in that** the relative amount of recombinant human acidic fibroblast growth factor in the gel is $2 \times 10^3$ to $8 \times 10^3$ IU/g.

7. The recombinant human acidic fibroblast growth factor (aFGF) gel according to claim 1, **characterized in that** the gel further contains a humectant and a preservative.

8. The recombinant human acidic fibroblast growth factor (aFGF) gel according to claim 6, **characterized in that** the humectant is selected from glycerols; and the preservative is selected from p-hydroxybenzoate preservatives.

9. The recombinant human acidic fibroblast growth factor (aFGF) gel according to claim 6, **characterized in that** the preservative is selected from one or more of methyl p-hydroxybenzoate, ethyl p-hydroxybenzoate, propyl p-hydroxybenzoate, and isopropyl p-hydroxybenzoate.

10. The recombinant human acidic fibroblast growth factor (aFGF) gel according to claim 6, **characterized in that** the mass of the preservative is 0.1 to 3‰ of the mass of the gel, preferably 0.2 to 1‰; and the mass of the humectant is 0.2 to 4% of the mass of the gel, preferably 0.5 to 2%.

## INTERNATIONAL SEARCH REPORT

| International application No. |
|---|
| **PCT/CN2024/070258** |

### A. CLASSIFICATION OF SUBJECT MATTER

A61K 9/06(2006.01)i; A61K 38/18(2006.01)i; A61K 47/18(2017.01)i; A61P 17/02(2006.01)i

According to International Patent Classification (IPC) or to both national classification and IPC

### B. FIELDS SEARCHED

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNABS, CNTXT, CJFD, CNKI, 万方学术数据库, WANFANG DATABASE, 读秀学术, DUXIU ACADEMIC, 百度, BAIDU, DWPI, VEN, WPABS, ENTXT, PubMed, Patentics, STNext: 重组人酸性成纤维细胞生长因子, 凝胶, 聚乙烯醇, 组氨酸, 肝素钠, 稳定, 保护剂, aFGF, a-FGF, rhAFGF, gel, Polyvinyl alcohol, PVA, Histidine, His, Heparin, Stabilizer, Protective agent, 腾瑞制药, 朱国兴, 付栋君, 唐芳, 关录凡, 李郑武, 赫佳鑫

### C. DOCUMENTS CONSIDERED TO BE RELEVANT

| Category* | Citation of document, with indication, where appropriate, of the relevant passages | Relevant to claim No. |
|---|---|---|
| PX | CN 116889545 A (SHANGHAI TENRY PHARMACEUTICAL CO., LTD.) 17 October 2023 (2023-10-17) <br> claims 1-10 | 1-10 |
| Y | CN 114010763 A (SHANGHAI TENRY PHARMACEUTICAL CO., LTD.) 08 February 2022 (2022-02-08) <br> description, paragraphs 7-18 and 26, and tables 1-9 | 1-10 |
| Y | CN 110063936 A (ANHUI XINHUAKUN BIOTECHNOLOGY CO., LTD.) 30 July 2019 (2019-07-30) <br> claim 3, and description, paragraph 21 | 1-10 |
| A | WO 2018232750 A1 (ZHUHAI ESSEX BIO-PHARMACEUTICAL CO., LTD.) 27 December 2018 (2018-12-27) <br> claims 4-12 | 1-10 |

☑ Further documents are listed in the continuation of Box C. ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **05 March 2024** | **13 March 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/070258** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2019231925 A1 (CHANGCHUN JA BIOTECH. CO., LTD. et al.) 01 August 2019 (2019-08-01)<br>    abstract | 1-10 |
| A | 邓新旺等 (DENG, Xinwang et al.). "肝素钠/聚乙烯醇复合水凝胶的制备与性能 (Preparation and Properties of Heparin Sodium/Polyvinyl Alcohol Composite Hydrogel)"<br>*应用化学 (Chinese Journal of Applied Chemistry)*,<br>Vol. 32, No. 12, 31 December 2015 (2015-12-31),<br>    pages 1358-1363 | 1-10 |
| A | 杨洪存等 (YANG, Hongcun et al.). "重组碱性成纤维细胞生长因子凝胶剂的制备 (Preparation of Recombinant Basic Fibroblast Growth Factor Gel)"<br>*中国生物制品学杂志 (Chinese Journal of Biologicals)*,<br>Vol. 22, No. 10, 31 October 2009 (2009-10-31),<br>    pages 1002-1004 | 1-10 |

Form PCT/ISA/210 (second sheet) (July 2022)

## INTERNATIONAL SEARCH REPORT
**Information on patent family members**

| International application No. |
| --- |
| **PCT/CN2024/070258** |

| Patent document cited in search report | | | Publication date (day/month/year) | Patent family member(s) | | | Publication date (day/month/year) |
| --- | --- | --- | --- | --- | --- | --- | --- |
| CN | 116889545 | A | 17 October 2023 | CN | 116889545 | B | 29 December 2023 |
| CN | 114010763 | A | 08 February 2022 | None | | | |
| CN | 110063936 | A | 30 July 2019 | None | | | |
| WO | 2018232750 | A1 | 27 December 2018 | KR | 20200021521 | A | 28 February 2020 |
| | | | | KR | 102480402 | B1 | 22 December 2022 |
| | | | | US | 2020157164 | A1 | 21 May 2020 |
| | | | | US | 11254723 | B2 | 22 February 2022 |
| | | | | JP | 2020533015 | A | 19 November 2020 |
| | | | | JP | 7044870 | B2 | 30 March 2022 |
| | | | | SG | 11201912799 | SA | 30 January 2020 |
| | | | | MY | 194351 | A | 29 November 2022 |
| | | | | EP | 3643784 | A1 | 29 April 2020 |
| | | | | EP | 3643784 | A4 | 05 May 2021 |
| US | 2019231925 | A1 | 01 August 2019 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111189805